# EUROPEAN PATENT APPLICATION

(11) **EP 4 563 077 A1**
(43) Date of publication of application: **04.06.2025**
(21) Application number: 24169905.7
(22) Date of filing: 12.04.2024
(51) Int. Cl.: A61B 5/00

(54) **HANDHELD DEVICE AND SYSTEM FOR OPTOACOUSTIC IMAGING**

(30) Priority: 28.11.2023 EP 23212807
(71) Applicant: iThera Medical GmbH, 81379 München (DE)
(72) Inventor: Leisching, Dr. Patrick, 81247 Munich (DE); Longo, Antonia, 80538 Munich (DE); Konradl, Josef, 85235 Odelzhausen (DE); Zahnd, Guillaume, 81379 Munich (DE)
(74) Representative: Linsmeier, Josef

(57) **Abstract**

The disclosure relates to a handheld device (1) and system for optoacoustic imaging of an object (3), the device (1) comprising a housing (2) configured to be grasped by an operator's hand and guided and/or positioned by the operator relative to the object (3) during imaging, wherein the following components are integrated in and/or provided on the housing: a) an illumination unit (4, 6) configured to illuminate the object (3) with time-varying electromagnetic radiation (5), the illumination unit (4, 6) comprising at least one radiation source (4, 6), in particular at least one laser diode (LD), vertical-cavity surface-emitting laser (VCSEL) or light-emitting diode (LED), having a front side (4a) and a back side (4b) opposite the front side and configured to emit the electromagnetic radiation (5) at the front side (4a) of the at least one radiation source (4), the illumination unit (4, 6) further comprising a first electronic controller (6) provided at the back side (4b) of and/or close to the at least one radiation source (4) and configured to control the at least one radiation source (4) to emit pulses of electromagnetic radiation (5) exhibiting at least one of a predefined electromagnetic spectrum and/or wavelength(s), pulse amplitude, pulse shape, pulse width (duration) and/or pulse repetition rate, and b) a detection unit (7, 9) configured to detect ultrasonic waves (8) emanating from the object (3) in response to illuminating the object (3) with the time-varying electromagnetic radiation (5), the detection unit (7, 9) comprising at least one ultrasonic transducer (7), in particular at least one micromachined ultrasonic transducer (MUT) and/or at least one piezoelectric ultrasonic transducer (PUT), having a front side (7a) and a back side (7b) opposite the front side and configured to convert ultrasonic waves (8) impinging at the front side (7a) of the at least one ultrasonic transducer (7) into detection signals, the detection unit (7, 9) further comprising a second electronic controller (9) provided at the back side (7b) of the at least one ultrasonic transducer (7) and configured to process the detection signals, in particular to digitize and/or filter the detection signals and to reconstruct an image of the object (3) based on the digitized and/or filtered detection signals.

## Description

The present disclosure relates to a handheld device and system for optoacoustic imaging according to the independent claims.

Currently, Point-of-Care (PoC) optoacoustic imaging (herein also referred to as "OA" or "OAI") devices are non-existent because current scanner technology is bulky, expensive, power consuming (>1000W) and complicated to operate. The state-of-the-art laser sub-component amounts for circa 50% of the total cost, size and power consumption. Laser technologies based on high power lasers (e.g., Nd:YAG+OPO), LEDs, or photodiodes have a low wall-plug efficiency and therefore require active cooling, consume a vast amount of energy, are bulky, make the examination room hot and noisy (air conditioning needed), and necessitate wearing safety goggles that are impractical for the clinician and uncomfortable for the patient. The electronics sub-system amounts for circa 25% of the total cost. Dedicated electronics (referred to as "DAQ" for "Data Acquisition device") with high throughput acquisition hardware are required to integrate the ultrasound and laser sub-components: triggering and transmission of the excitation signals, reception of the generated (OA) and reflected ultrasonic (herein also referred to as "US") acoustic pressure waves. Additionally, a high-performance graphic card is needed to reconstruct high quality OA images in real-time. The piezoelectric (i.e., ceramics, crystals, polymers and piezo-composite) ultrasound transducers sub-component amounts for circa 15% of the total cost.

It is an object of the present disclosure to provide a handheld device and system for opto-acoustic imaging which at least partially overcome at least one of the above-mentioned limitations.

The object is achieved by a handheld device and system for optoacoustic imaging according to the independent claims.

According to a first aspect of present disclosure, a handheld device for optoacoustic imaging of an object comprises a housing configured to be grasped by an operator's hand and to be guided and/or positioned by the operator relative to the object during imaging, wherein an illumination unit and a detection unit are integrated in and/or provided on the housing. The illumination unit is configured to illuminate the object with time-varying electromagnetic radiation and comprises at least one radiation source, in particular at least one laser diode (LD), in particular at least one vertical-cavity surface-emitting laser (VCSEL), or at least one light-emitting diode (LED), having a front side and a back side, which is opposite the front side, and being configured to emit the electromagnetic radiation at the front side of the at least one radiation source. The illumination unit further comprises a first electronic controller provided at the back side of and/or close to the at least one radiation source and configured to control the at least one radiation source to emit pulses of electromagnetic radiation exhibiting at least one of a predefined electromagnetic spectrum and/or wavelength(s), pulse amplitude, pulse shape, pulse width (duration) and/or pulse repetition rate. The detection unit is configured to detect ultrasonic waves emanating from the object in response to illuminating the object with the time-varying electromagnetic radiation and comprises at least one ultrasonic transducer, in particular at least one micromachined ultrasonic transducer (MUT) and/or at least one piezoelectric ultrasonic transducer (PUT), having a front side and a back side opposite the front side and being configured to convert ultrasonic waves impinging at the front side of the at least one ultrasonic transducer into detection signals, in particular a time series of electrical signals. The detection unit further comprises a second electronic controller provided at the back side of the at least one ultrasonic transducer and configured to process the detection signals, in particular to digitize and/or filter the detection signals and to reconstruct an image of the object based on the digitized and/or filtered detection signals.

According to a second aspect of present disclosure, a system for optoacoustic imaging of an object comprises a handheld device according to the first aspect, and a computer system, in particular a mobile computer device and/or a computer workstation and/or a cloud computing system, located outside the handheld device, in particular outside the housing of the handheld device, and being in a data communication with the first and/or second electronic controller, the computer system being configured to further process the reconstructed image.

Preferred aspects of present disclosure are based on the approach to provide a handheld device and system for optoacoustic imaging, wherein most, preferably all, of the components for object illumination, for controlling the illumination, for ultrasonic wave detection, and for processing the detection signals are integrated in a single handheld probe. This is preferably achieved by replacing bulky standard laser sources (and related electronics) by semiconductor lasers, such as laser diodes (LDs), in particular vertical-cavity surface-emitting lasers (VCSELs), and/or light-emitting diodes (LEDs), and by integrating the electronics for controlling the laser source at the backside of and/or close to the laser source (generally referred to as "backside electronics") to achieve short pulses (having a duration preferably between 1 and 100 ns) of the laser source. In this way, the so-called wall-plug efficiency can be increased to more than 50%, while heat dissipation in the handheld probe during operation can be reduced to less than 10 W. Further, according to a preferred design option, bulky piezoelectric ultrasound transducers (and related electronics) according to the prior art are replaced by micromachined ultrasonic transducers (MUTs), in particular capacitive micromachined ultrasonic transducers (CMUTs) and/or piezoelectric micromachined ultrasonic transducers (PMUTs), and the electronics for controlling the MUT(s) and/or for detection signal processing (e.g. signal digitalization, filtering, basic image reconstruction and processing) is integrated at the backside of the MUTs (also referred to as "backside electronics"), so that the MUTs and the electronic circuit are integrated on the same chip. According to a preferred alternative design option, the detection unit comprises one or more piezoelectric ultrasonic transducers (PUTs), wherein the electronics for controlling the PUT(s) and/or for detection signal processing (e.g. signal digitalization, filtering, basic image reconstruction and processing) is integrated at the backside of the PUT(s) (also referred to as "backside electronics"), so that the PUT(s) and the electronic circuit are integrated on the same chip.

Combining semiconductor laser(s) for object illumination, MUT(s) and/or PUT(s) for ultrasonic wave detection and backside electronics for controlling the laser source(s) and processing the detection signals from the MUT(s) or PUT(s), respectively, allows for a particularly compact integration of these components in and/or on the housing of the handheld probe.

As a result, the handheld device and system according to present disclosure are less bulky, less expensive, less power consuming and less complicated to operate compared to state-of-the-art devices and, therefore, particularly suitable for optoacoustic imaging in a point-of-care (PoC) environment.

Preferably, the at least one radiation source is and/or comprises a vertical-cavity surface-emitting laser (VCSEL). Within the context of present disclosure, a VCSEL preferably relates to a type of semiconductor laser diode with laser beam emission perpendicular from the top surface, which is also referred to as "front side" herein.

Preferably, the at least one laser diode (LD), in particular vertical-cavity surface-emitting laser (VCSEL) has a substrate and is mounted on a sub-mount which may be attached to a heat-dissipating holder, e.g. a metal block such as a copper block.

Alternatively or additionally, the at least one radiation source is and/or comprises at least one light-emitting diode (LED) which has a substrate and is mounted on a sub-mount which may be attached to a heat-dissipating holder, e.g. a metal block such as a copper block.

Preferably, the metal and/or copper block can be provided with heat pipes to further improve heat dissipation.

The first electronic controller, e.g. a printed circuit board (PCB) provided with appropriate electronic components, can be provided at the back side of the laser source(s) to achieve a particularly short distance between the electronic components and the laser source(s). Alternatively, the first electronic controller does not necessarily have to be provided at the back side of the laser source(s) in the narrower sense, i.e. opposite the front side of the laser source(s), but can rather be provided in any other way close to the laser source(s) so that short distances, preferably smaller than 10 mm (10-20ns), more preferably smaller than 5 mm, in particular smaller than 500µm (<1 ns), between the electronic components of the first electronic controller and the laser source(s) are achieved. It is important to note that, for the sake of simplification, both alternatives are generally referred to herein as "backside electronics" which, therefore, encompasses any arrangement of the first electronic controller at the back side of the laser source(s) in the narrower sense and/or close to the laser source(s) and/or close to the back side of the laser source(s).

For example, the laser sources, in particular VCSELs, can be mounted on a top surface of a cuboidal or brick-shaped metal block, preferably copper block, serving as a heat sink, whereas the PCB of the first electronic controller can be provided at a lateral surface, which runs perpendicularly to the top surface, of the copper block. Preferably, the metal and/or copper block can be provided with heat pipes to further improve heat dissipation.

Preferably, the laser sources are connected to the components of first electronic controller via bonded gold wires to achieve both good electric coupling and good thermal management. Preferably, the first electronic controller comprises an integrated circuit based on complementary metal-oxide-semiconductor (CMOS, Si or GaN) technology and is mounted, in particular by flip-chip bonding, on the back side (< 500 µum) of and/or close (wire bonding) to the at least one radiation source in such a way that electrical connections between the integrated circuit and the at least one radiation source are shorter than 10mm. In this way, a particularly fast control, in particular switching, of the laser source, in particular the VCSEL, is achieved so that particularly short pulses (i.e. pulses with particularly narrow pulse width) and/or series of pulses with a particularly high pulse repetition rate can be generated.

Preferably, the first electronic controller is configured to control the at least one radiation source to emit pulses of electromagnetic radiation exhibiting a pulse width (duration) between 1 ns and 100 ns and/or a repetition rate between 100 Hz and 10 kHz.

Preferably, the first electronic controller is configured to trigger, i.e. initiate and/or cause and/or activate, the at least one radiation source, in particular the at least one VCSEL, to emit the pulses of electromagnetic radiation. While in conventional devices and systems using, e.g., YAG-OPO lasers the emission of the excitation pulses triggers the data acquisition electronics (DAQ), i.e. the YAG-OPO shot triggers the DAQ, it is preferred that in the disclosed device and/or system the first electronic controller and/or DAQ triggers the at least one radiation source, in particular the at least one VCSEL.

Preferably, the at least one micromachined ultrasonic transducer is a capacitive micromachined ultrasonic transducer (CMUT) and/or piezoelectric micromachined ultrasonic transducer (PMUT). Preferably, a CMUT is configured to receive ultrasonic waves by measuring a change in capacitance by means of a construction (silicon substrate layer complemented by a metallized layer) which functions similarly to electrodes. Advantageously, CMUTs have compact form factors, allow for higher imaging resolutions, high bandwidths and higher sensitivity. Therefore, providing the detection unit with one or more CMUTs allows for optoacoustic imaging at lower cost and in a particularly small package within the handheld probe. Preferably, a PMUT configured to receive ultrasonic waves by detecting flexural vibrations which are measured by a piezoelectric membrane. Though it still utilizes a piezoelectric film, PMUT arrays offer substantial benefits over probes that rely on conventional piezoelectric crystals or ceramics, namely enhanced 3D imaging via advanced resonance frequency and high reliability. Advantageously, PMUTs offer a higher capacitance and lower electrical impedance over CMUTs to increase probe sensitivity by reducing the impact of parasitic capacitance and enabling the use of low voltage electronics. On the other hand, under certain conditions and/or depending on the particular application of the device or system, CMUTs may yield advantages and, therefore, be preferred over PMUTs.

For example, it may be preferred that at least one PMUT for creating ultrasonic waves (to be reflected by the object to obtain ultrasound images) is provided in combination with at least one CMUT for detecting ultrasonic waves which are reflected by the object in response to the impinging ultrasonic waves created by the at least one PMUT and/or emitted by the object in response to illuminating the object with the pulsed electromagnetic radiation.

Preferably, the at least one ultrasonic transducer, in particular the piezoelectric ultrasonic transducer (PUT) and/or micromachined ultrasonic transducer (MUT), in particular capacitive micromachined ultrasonic transducer (CMUT) and/or piezoelectric micromachined ultrasonic transducer (PMUT), has a substrate and is mounted on a sub-mount, which may be attached to a, in particular heat-dissipating, holder, e.g. a metal block such as a copper block. Preferably, the metal and/or copper block can be provided with heat pipes to further improve heat dissipation. For example, the second electronic controller does not necessarily have to be provided at the backside of the PUT(s) or MUT(s), respectively, in the narrower sense, i.e. opposite the front side of the PUT(s) or MUT(s), but can rather be provided in any other way close to the PUT(s) or MUT(s) so that short distances, preferably smaller than 10 mm, more preferably smaller than 5 mm, in particular smaller than 500µm, between the electronic components of the second electronic controller and the PUT(s) or MUT(s) are achieved. While these distances do not necessarily have to be met in applications where ultrasound frequencies up to 10 MHz are to be detected (i.e. here, the distances between the second electronic controller and the PUT(s) or MUT(s) can be considerably longer), providing short distances as specified above is particularly advantageous in applications where ultrasound frequencies of 10 MHz or more are to be detected.

Preferably, the device further comprises at least one sensor unit configured to detect whether the handheld device is in contact with and/or close to the object, wherein the first electronic controller is configured to control the at least one radiation source to emit the pulses of electromagnetic radiation only if the at least one sensor unit detects that the handheld device is in contact with or close to the object, and/or to stop emitting pulses of electromagnetic radiation if the at least one sensor unit detects that the handheld device is not in contact with or not close to the object. Providing the handheld device with the at least one sensor unit makes the device compliant with laser safety regulations and allows for avoiding the use of goggles.

Preferably, the at least one sensor unit comprises a stray light sensor, in particular a photodiode, configured to detect stray light emanating from the object in response to illuminating the object with the time-varying electromagnetic radiation, wherein detecting the stray light is indicative that the handheld device is in contact with and/or close to the object, whereas not detecting stray light is indicative that the handheld device is not in contact with and/or not close to the object. For example, the at least one sensor can be a photodiode or the like which detects the stray light. Since, in this embodiment, the stray light corresponds to a part of the electromagnetic radiation emitted by the at least one radiation source towards the object, no further radiation source is necessary.

Alternatively or additionally, the at least one sensor unit comprises at least one contact sensor configured to detect whether the handheld device is in contact with the object, wherein the at least one contact sensor comprises at least one of the following: an optical sensor, a conductivity sensor, a thermocouple, an ultrasonic sensor, an accelerometer sensor, a resistive and/or capacitive touch screen and/or a piezoelectric sensor.

Preferably, the at least one sensor unit comprises both the stray light sensor and the contact sensor, wherein the first electronic controller is configured to control the at least one radiation source to emit the pulses of electromagnetic radiation only if both the stray light sensor and the contact sensor detect that the handheld device is in contact with and/or close to the object, and/or to stop emitting pulses of electromagnetic radiation if at least one of the stray light sensor and/or the contact sensor detects that the handheld device is not in contact with or not close to the object. In this for embodiment of a laser safety mechanism, two factors are combined: a) the laser-induced stray light reflected by the object, in particular the skin of a patient, is measured by a, e.g., diode photosensor, and the at least one laser source is turned off automatically when no such light arrives as this implies that contact to the object, in particular the skin, has been lost. b) the, preferably mechanical, contact sensor triggers laser inhibition when the probe surface is not in contact with an object. In this way, compliance with laser safety regulations can be achieved in a particularly reliable manner. Further, the use of goggles is completely dispensable.

Preferably, the handheld device further comprises a calibration unit comprising at least one calibration sensor configured to detect a reference signal, in particular electromagnetic radiation reflected and/or scattered by the imaged object and/or by a reference object and/or ultrasonic waves emanating from the imaged object and/or a reference object, and a calibration controller configured to derive a calibration signal from the detected reference signal, wherein a) the first electronic controller is configured to control the at least one radiation source by considering the calibration signal and/or b) the second electronic controller is configured to control the at least one ultrasonic transducer and/or to process the detection signals by considering the calibration signal. Preferably, the at least one calibration sensor comprises and/or is given by a stray light sensor, in particular a photodiode, which is configured to detect, as the reference signal, stray light emanating from the imaged object and/or from a reference object in response to illuminating the imaged object or reference object, respectively, with the time-varying electromagnetic radiation. Alternatively or additionally, it is preferred that the at least one calibration sensor comprises and/or is given by at least one ultrasonic transducer of the detection unit and configured to detect, as the reference signal, ultrasonic waves emanating from the imaged object and/or a reference object in response to illuminating the imaged object or reference object, respectively, with the time-varying electromagnetic radiation. In these preferred embodiments, an OA and/or US calibration unit is integrated in the handheld probe. Preferably, a calibration of the device can be achieved by directly using the VCSELs' ability to sense the light which has been reflected and/or scattered by the imaged object, which greatly simplifies conventional calibration procedures. Alternatively or additionally to measuring the energy or intensity of the light directly in the optical pathway (e.g. with a separate light sensor), the energy or intensity of the light can be measured indirectly but more comprehensively in the optoacoustic pathway (by detecting the mechanical pressure waves induced by the light). By automatically and/or regularly performing such a calibration and/or checking the resulting calibration signals the following advantages are achieved: It can be checked and/or ensured that the device and/or system is stable in time, without a degradation and/or increase of transmitted light. Further, in case of two or more different devices and/or systems an intensity scaling in the measured data can be made, so that the results are quantitatively comparable although the measured data was generated by different devices or systems, respectively.

Preferably, the handheld device further comprises an interface unit configured to establish a, preferably wireless, data communication between, on the one hand, the first and/or second electronic controller and, on the other hand, a computer system, in particular a mobile computer device and/or a computer workstation and/or a cloud computing system, located outside the handheld device and configured to further process the reconstructed image. In this way, advanced data processing for better image quality and quantification is possible without enlarging the size of the handheld device itself, because the latter is just provided with a connectivity to a, for example cloud-based, high performance computing unit.

Preferably, a pre-amplifier is provided to amplify the signals generated by the detection unit, in particular by the at least one PUT and/or MUT, prior to entering and/or being processed in the second electronic controller. This is particularly advantageous to achieve a high signal-to-noise ratio, in particular when using VCSELs enabling high repetition rates. A high signal-to-noise-ratio allows for a higher maximum depth penetration (and image quality) in the tissue.

Preferably, the first electronic controller is configured to control the at least one radiation source to, in particular simultaneously, emit at least two different time series patterns of electromagnetic radiation, wherein the at least two time series patterns differ from each other with respect to at least one of the following: amplitude, shape, duration and/or repetition rate, the at least one ultrasonic transducer is configured to detect the ultrasonic waves emanating from the object in response to, in particular simultaneously, illuminating the object with the at least two different time series patterns of electromagnetic radiation, to convert the detected ultrasonic waves into detection signals, and to separate the detection signals into at least two separate detection signals, the at least two separate detection signals characterizing the response of the object to each of the at least two different time series patterns of electromagnetic radiation, and the second electronic controller is configured to separately process the at least two separate detection signals, in particular to reconstruct at least two different images of the object based on the at least two separate detection signals. Illuminating the object to be imaged with different time series patterns of electromagnetic radiation, in particular at least two different series of pulses exhibiting a different electromagnetic spectrum and/or wavelength and/or pulse amplitude and/or pulse shape and/or pulse width (duration) and/or pulse repetition rate and/or pulse polarization and/or pulse phase, is also referred to as "encoding" and allows for a fast, in particular simultaneous, and reliable acquisition of ultrasonic waves relating to different components of and/or different depths within the object to be imaged.

Preferably, the ultrasonic transducer has a frequency-dependent sensitivity having a transducer bandwidth (preferably a broadband) within which the ultrasonic transducer is sensitive to ultrasonic waves, the transducer bandwidth preferably including a center frequency region in which the sensitivity to ultrasonic waves is maximum, and the first electronic controller is configured to tune, by controlling the at least one radiation source, the pulse width of the emitted pulses of electromagnetic radiation such that the resulting ultrasonic waves emanating from the object in response to illuminating the object with the emitted pulses of electromagnetic radiation exhibit at least one frequency bandwidth which matches with and/or falls within the transducer bandwidth. In this way, the laser pulse can be optimized to make the device or system response fit to the (given) transducer bandwidth, so that the physically maximum achievable signal can be achieved. Preferably, the emitted laser pulse can be optimized to make the device or system frequency response precisely match the specific receiving bandwidth of the acoustic transducer, so that the physically maximum information content contained in the signal can be recorded. As further explained in detail below, the frequency response of the signal can be modified to match the needs (namely, that the signal bandwidth corresponds to that of the receiving ultrasonic sensor/transducer) by preferably changing the temporal duration (width) of the illumination pulse(s).

Preferably, the ultrasonic waves emanating from the object in response to illuminating the object with the emitted pulses of electromagnetic radiation exhibit a first frequency bandwidth at a fundamental frequency and a second frequency bandwidth at a, in particular second, harmonic frequency of the fundamental frequency, wherein the transducer bandwidth is configured and/or configurable to be sensitive to both at least a part of the first frequency bandwidth at the fundamental frequency and at least a part of the second frequency bandwidth at the, in particular second, harmonic frequency of the fundamental frequency. Alternatively or additionally, the first electronic controller is configured to tune, by controlling the at least one radiation source, the pulse width of the emitted pulses of electromagnetic radiation such that the resulting ultrasonic waves emanating from the object in response to illuminating the object with the emitted pulses of electromagnetic radiation exhibit a first frequency bandwidth at a fundamental frequency and a second frequency bandwidth at a, in particular second, harmonic frequency of the fundamental frequency, wherein both the fundamental frequency and the harmonic frequency matches with and/or falls within the transducer bandwidth of the ultrasonic transducer. The device further comprises at least one filter, in particular bandpass filter, which is preferably configured to separate the detection signals, which were obtained by detecting the ultrasonic waves emanating from the object in response to illuminating the object with the emitted pulses of electromagnetic radiation exhibiting the first frequency bandwidth at the fundamental frequency and the second frequency bandwidth at the harmonic frequency, into first detection signals characterizing the ultrasonic waves exhibiting the first frequency bandwidth at the fundamental frequency, and second detection signals characterizing the ultrasonic waves exhibiting the second frequency bandwidth at the harmonic frequency. Further, the second electronic controller is preferably configured to separately process the first and second detection signals, in particular to reconstruct two different images of the object based on the first and the second detection signals. In this way of illuminating the object and/or detecting ultrasonic waves in response to illuminating the object, which is also referred to as "second harmonic imaging", the bandwidth of the excitation signal (e.g. by using custom pulses with VCSELs) is matched to the bandwidth of the receiving sensors (e.g. by using custom PMUTs/CMUTs), so that not only the fundamental frequency (referred to as "first harmonic") but also the second harmonic can be recorded. While this technique can be used in conventional pulse echo ultrasound, it is currently not used in OA devices because the amplitude of the second harmonic is comparable to noise-level. In a second step, a simple bandpass filter is applied to either the first or the second harmonic to isolate a specific frequency band. The first harmonic will provide a bulky image, and the second harmonic will greatly enhance the details and have a greater spatial resolution due to the contribution of high frequencies.

Preferably, the illumination unit comprises a plurality of vertical-cavity surface-emitting lasers (VCSELs) and/or the detection unit comprises a plurality of ultrasonic transducers, in particular piezoelectric ultrasonic transducers (PUTs) and/or micromachined ultrasonic transducers (MUTs), in particular capacitive micromachined ultrasonic transducers (CMUTs) and/or piezoelectric micromachined ultrasonic transducers (PMUTs) and/or a combination of both, and wherein at least one of the following applies: i) the VCSELs are arranged in at least one one-dimensional or two dimensional array, in particular the VCSELs can be arranged in any arbitrary line or grid, ii) the VCSELs are configured to emit electromagnetic radiation exhibiting only one electromagnetic spectrum and/or wavelength or to emit electromagnetic radiation exhibiting at least two different electromagnetic spectra and/or wavelengths, iii) the VCSELs are arranged on one side or both sides of the MUTs in an elevational direction, iv) the VCSELs are configured and/or arranged to deliver the electromagnetic radiation obliquely at any angle (configuration of dark field illumination, v) an optical mirror and/or lenses is or are provided to redirect the beam of electromagnetic radiation on the MUTs' principal acoustic axis (configuration of bright field illumination), vi) the MUTs and VCSELs are arranged on the same plane in a matrix with a specific regular pattern (configuration of semi-bright field illumination), vii) the VCSELs are centered in the MUTs (through-hole illumination or bright field illumination), viii) in case of transparent MUTs, VCSELs are coaxially aligned with the MUTs, so that the illumination of the object occurs via and/or through the MUTs (configuration of bright field illumination). By at least one of the aforementioned preferred embodiments, or a combination thereof, the device and/or system is further improved in view of specific imaging applications.

In summary, preferred aspects of present disclosure are based on the approach to provide a PoC optoacoustic device that integrates, preferably all, optical, acoustic, and electronic components in a single handheld probe. In preferred embodiments, at least one of the following technical measures is or are provided:
1. Replacing standard laser sources (and related electronics) with Vertical-Cavity Surface-Emitting Lasers (VCSELs) and backside integrated ToF (time-of-flight) electronics with high wall-plug efficiency of >50%, to lower the heat dissipation in the handheld during operation below 10W.

ToF electronics is an established technology, in particular in industrial fields such as automotive, robotics and smartphones, and is traditionally used in so-called "ToF cameras" to measure the distance to an object, wherein the underlying principle is to measure the time for the round-trip of a light beam reflected on an object. Aspects of ToF electronics are preferably used and/or exploited in the context of present disclosure (optoacoustic imaging). ToF technology is already industry-mature and available "out-of-the-shelf" which enhances its feasibility for optoacoustic imaging applications. Further, ToF technology is already miniaturized (less than 10 grams), require only very limited amount of energy to operate (wall-plug efficiency >50%, heat dissipation <10W), and can be integrated with other (custom) sub-components. This results in advantages over current technology because it crucially enables to integrate an ensemble of sub-components directly in the handheld probe (extremely reduced form-factor without generating heat that would damage the components and/or hurt the user). Further, ToF technology is capable to generate nearly arbitrary pulse shapes (not only squares, but also triangles or Gaussian curves), of very short duration (below 100ns), at a very fine gating temporal resolution (order of the 100 picoseconds). This results in advantages over current technology because it allows for using an optimal illumination pattern, in terms of pulse shape and duration, to obtain the most favorable ultrasonic signal wavelength. This bandwidth adaptation can be preferably tailored to target both the absorption spectrum of target molecules, as well as the physical properties of the ultrasonic recording device.

Preferably, ToF electronics is exploited and/or implemented as a sub-component of the optoacoustic handheld device. A typical ToF device is composed of five major elements (master electronics, optical lens, illumination source, light sensor, computation unit). Preferably, only the master electronics is used in the handheld device and/or a system, while the other parts can be replaced for specific needs.
(a) Preferably, the master electronics of a ToF device is used as the first electronic controller to control the pulse modulation, length, magnitude, shape, and repetition rate. This sub-component offers technical possibilities, namely generation of nearly-arbitrary pulse shapes, at high accuracy, with minimal energy input, that are not achievable with conventional optoacoustic systems. Preferably, the master electronics can be integrated in combination with several other custom sub-components. Depending on the specific purpose of an application of the device and/or system, the four remaining components of a typical ToF electronics will be either modified or ignored (further details as to that are given below).
(b) Illumination source: Typically, LED or edge emitting laser diodes can be used. Rather, for the handheld device and/or system preferably VCSELs are used.
(c) Light sensor: Typically used to record reflected light. Preferably, the ToF light sensor can be ignored, since optoacoustic imaging records reflected ultrasound pressure waves. Preferably, for the handheld device and/or system MEMs/PMUTs/CMUTs and/or ceramic piezos are used.
(d) Optical lens (can be omitted in preferred embodiments): Preferably, the outgoing optical part will be tailored together as part as the VCSELs sub-component (b) and the incoming acoustic part with be tailored as part as the MEMs sub-component (c).

Optionally, an acoustic lens can be provided on the MUT(s), in particular on the PMUT(s), to compensate for curved/non-curved designs.
(e) Computation unit (can be omitted in preferred embodiments): Preferably, the incoming pressure wave will be recorded, pre-amplified and/or averaged and/or converted from analog to digital and/or decoded, and signals will be sent to the main body of the optoacoustic scanner for processing (namely, image reconstruction).

2. Increasing the repetition rate from 25Hz to 100Hz - 10KHz will allow for burst mode, modulation with codes and respectively additional gain of 3-6dB in the signal-to-noise ratio thus allowing to operate the device and/or system with lower energy per pulse.

Preferably, at least one intensity-modulated light source is used. Advantageously, intensity-modulated light sources represent an affordable alternative to short-pulsed laser. Preferably, at least one of the following intensity modulation methods is or are applied: Sinusoidal/continuous Wave (CW) excitation, chirped/frequency sweep modulation methods, sinusoidal burst, and/or tone burst consisting of a train of equally-delayed short pulses. The intensity of the OA signal is inversely proportional to the number of pulses in the cycle. Further, the bandwidth of the OA signal is always lower or equal to the one generated by pulsed laser, which is beneficial for limited bandwidth transducer(s), allows for a high sensitivity and strong noise rejection because noise is approximately proportional to the square root of the bandwidth, and allows for optimizing the sensitivity in the band of interest. Further, the maximum optical energy delivered into skin can be higher than a single pulse, whereby the SNR can be increased.

Preferably, so-called orthogonal encoding is performed. Analyzing the ultrasonic waves in the Fourier domain (FD) can also enable concurrent illumination at multiple wavelengths, by modulating sources of different color at different frequencies. The wavelengths appear then at different discrete frequency in frequency domain.

3. Using the potential of a time-of-flight (ToF) electronics from automotive and telecommunication, the pulse width can be tuned from several ns to 100ns thus allowing an optimized excitation depending on the penetration depth and minimum object size.

4. For a given transducer bandwidth, the laser pulse can be optimized to make the system response fit to this bandwidth, so the physically maximum achievable signal can be realized. Preferably, the emitted laser pulse can be optimized to make the system frequency response precisely match the specific receiving bandwidth of the acoustic transducer, so the physically maximum information content contained in the signal can be recorded. Preferably, the frequency response of the signal can be modified to match the needs (namely, that the signal bandwidth corresponds to that of the receiving ultrasonic sensor) by changing the temporal duration (width) of the illumination pulse.

5. Replacing bulky piezos (and related electronics) with microelectromechanical systems (MEMS), more specifically Capacitive Micromachined Ultrasonic Transducers (CMUTs) and Piezoelectric Micromachined Ultrasonic Transducers (PMUTs), including transparent versions or combinations of them. It is preferred that to utilize CMUT(s), PMUT(s), and/or a combination thereof (instead of just one type, e.g., one for receiver, one for transmitter). Alternatively, piezoelectric ultrasonic transducers (PUTs) with backside electronics can be used for detecting ultrasonic waves.

Optionally, an acoustic lens can be provided on the xMUT(s) for compensation of curvature of the bulk piezos.

6. Integrating MEMS and electronic circuits on the same chip, including circuits for computing on the chip (basic functionalities such as signal digitalization, filtering, basic image reconstruction and processing).

7. Integrating VCSELs, MEMS and entire electronics (i.e., laser controller (also referred to as "first electronic controller"), OA/US mode controller, front-end computing unit (also referred to as "second electronic controller"), power supply, etc.) within the housing of the handheld probe using backside electronics.

8. Replacing the scanner reconstruction unit, control panel, and display by a lightweight, e.g. a touchpad-like, computing device (e.g., laptop, tablet, phone). This is achieved by integrating part of the necessary processing directly in the handheld device, thanks to the so-called backside electronics that is or are small and efficient enough to be directly adjacent to the optical sources and acoustic receivers. This way, the scanner body shall only focus on image reconstruction tasks. Furthermore, the algorithms running on the scanner body will be computationally optimized so they are more frugal and necessitate less computational resources.

9. Integrating connectivity to a remote computer workstation and/or a cloud-based high performance computing unit for advanced data processing toward better image quality and quantification. Here the term "cloud" denotes any computing instrument hosted by a third party or hosted by the provider and/or manufacturer of the handheld device or system, or simply being a special standalone workstation preconfigured by the provider and/or manufacturer of the handheld device or system on the desk of the user (similarly as an MRI system comprised of a scanner and a corresponding specific computer).

10. Including a control/sensor unit on the handheld device to detect probe contact with tissue for two currently non existing benefits: laser safety regulation and avoid use of goggles. This safety mechanism can be based on a combination of two factors: 1) The laser-induced stray light reflected on the skin is always measured by a diode photosensor, and the laser is turned off automatically when no such light arrives as this implies that contact to the skin has been lost. 2) A mechanical contact sensor triggers laser inhibition when the probe surface is not in contact with an object.

11. Integrating an OA/US calibration unit on the handheld probe. Preferably, calibration is performed by directly using the VCSELs ability to sense the reflected light, which greatly simplifies the conventional procedure. Calibration of the light energy is relevant, and this can be measured directly in the optical pathway (by a light sensor), or indirectly but more comprehensively in the opto-acoustic pathway (reception of mechanical pressure wave induced by laser light). The incentive of these automated and regular checks is two-fold: 1) ensure that a system is stable in time, without degradation or increase of transmitted light, and 2) assess two different systems and make the necessary intensity scaling in the measured data so the results can be quantitatively comparable.

These implementations, taken alone or by combining at least two of these implementations, will lead to miniaturization of the handheld device, reduction in cost and power consumption, increased safety (laser class of device from 4 to 1c, i.e. laser is class 3 or 4 but two independent security mechanisms switch the system off within the time frame for one emitted pulse. Mechanism 1 is contact control, mechanism 2 is the measurement of the backscattered light from the skin), control of system stability for optimal image quality and system performance at each imaging session, and improved image quality.

In particular, it is pointed out that the prior art fails to disclose at least one of the following preferred aspects of present disclosure: VCSEL(s) as source for modulated light with wavelength encoding, VCSEL(s) with modulated light combination with xMUTs, different light excitation shape and scheme (herein also referred to as "encoding"), OA imaging using second harmonic for ultrasound transducer, and/or using ToF electronics to control the VCSEL(s).

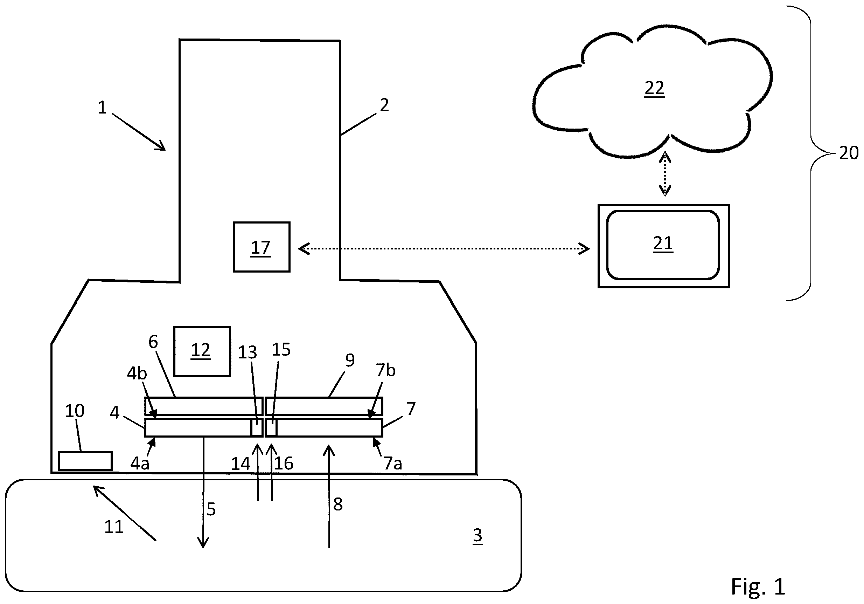

Preferably, the present disclosure differs from and/or is advantageous over the prior art with respect to at least one of the following preferred aspects:
- Combination of VCSELs and MEMs in a general-purpose imaging probe.
- Integration of VCSEL and electronics on same chip for OA applications. For details, see the description below with reference to the example shown in figure 1.
- Integration of calibration unit in OA/US PoC devices.
- Integration of laser safety unit on OA PoC devices.
- Integration of data flow and processing from OA handheld to high performance computing (preferably to cloud computing).
- Tunability of the laser pulse length/shape in agreement with transducer central frequency.
- Burst mode and/or code gain implementation for OA.
- Second harmonic imaging in OA devices. By matching the bandwidth of the excitation signal (using custom pulses with VCSELs) to the bandwidth of the receiving sensors (using custom PMUTs/CMUTs), the fundamental frequency (first harmonic) but also the second harmonic can be recorded. This technique can be used in traditional ultrasound but is currently not used in OA devices because the amplitude of the second harmonic is comparable to noise-level. In a second step, a simple bandpass filter and be applied to either the first or the second harmonic to isolate a specific frequency band. The first harmonic will provide a bulky image, and the second harmonic will greatly enhance the details and have a greater spatial resolution due to the contribution of high frequencies. For further details, it is referred to Gao X, Chen X, Hu H, Wang X, Yue W, Mu J, Lou Z, Zhang R, Shi K, Chen X, Lin M. A photoacoustic patch for three-dimensional imaging of hemoglobin and core temperature. Nature Communications. 2022 Dec 15;13(1):7757, which is incorporated by reference herein.

The device and system according to aspects of present disclosure is particularly suited and/or advantageous in the field of sensing and/or imaging of perfusion and/or oxygenation in biological tissue using (only) two different illumination wavelengths, for example at 680/850nm nm or 760/850 nm (measuring of prefusion/oxygenation). Other implementations may require only one wavelength (e.g. 805nm when imaging of vessels in tissue) and/or longer wavelengths (imaging of collagen at 850/960/1060nm).

Further advantages, preferred and/or optional and/or alternative features, aspects and examples of the present disclosure will be apparent from the following description and figures showing
- Fig. 1: a schematic representation of a cross-sectional view of an example of a device and system for optoacoustic imaging;
- Fig. 2: examples of frequency responses of ultrasound transducer(s) in response to an illumination of an object with pulses of electromagnetic radiation exhibiting different pulse durations;
- Fig. 3: a diagram (upper part) showing an example of a frequency response of an ultra-sound transducer at a first and second harmonic frequency, and optoacoustic images (lower part) which where separately reconstructed therefrom;
- Fig. 4: a top view (left part) and side view (right part) of an example of a two-dimensional arrangement of VCSELs;
- Fig. 5: a side view (upper part) and top view (lower part) of examples of different arrangements of VCSELs and MUTs;
- Fig. 6: a comparison of a system for optoacoustic imaging according to the prior art (left) with a system according to the present disclosure (right) in a schematic representation;
- Fig. 7: a perspective view of another example of a device and system for optoacoustic imaging;
- Fig. 8: a schematic representation of a circuit diagram of an example of the illumination unit; and
- Fig. 9: a perspective view of yet another example of a device and system for optoacoustic imaging.

Figure 1 shows a cross-sectional view of an example of a device 1 and system for optoacoustic imaging. The device 1 comprises a housing 2 having a size and shape such that it can be grasped by an operator's hand (not shown) and guided and/or positioned by the operator relative to an object 3 to be imaged. An illumination unit 4, 6 is integrated in the housing 2 and configured to illuminate the object 3 with a time-varying electromagnetic radiation 5. The illumination unit 4, 6 comprises at least one radiation source 4, preferably at least one vertical-cavity surface-emitting laser (VCSEL), having a front side 4a and a back side 4b opposite the front side 4a and being configured to emit the electromagnetic radiation 5 at the front side 4a towards the object 3.

A first electronic controller 6 is provided at the back side 4b of the radiation source 4 and configured to control the radiation source 4 to emit pulses of electromagnetic radiation exhibiting a predefined electromagnetic spectrum, pulse amplitude, pulse shape and/or pulse width (duration) and/or pulse repetition rate. Preferably, the first electronic controller 6 comprises an integrated circuit (IC) based on complementary metal-oxide-semiconductor (CMOS) technology and is mounted, in particular by flip-chip bonding, on the back side 4b of the at least one radiation source 4, in particular the at least one VCSEL, in such a way that electrical connections between the integrated circuit and the at least one radiation source 4 are shorter than 500 µm (<1 ns) or <10mm (<10-20ns). Alternatively or additionally, the first electronic controller 6 is configured to control the at least one radiation source 4, in particular the at least one VCSEL, to emit pulses of electromagnetic radiation exhibiting a pulse width (duration) between 1 ns and 100 ns and/or a repetition rate between 100 Hz and 10 kHz.

Configuring the first electronic controller 6 as "backside electronics" as described above is particularly preferred, e.g., when a single VCSEL (having a typical size of approx. 40 × 40 µm²) or a small VCSEL array (having a typical size of approx. 1 × 1 mm²) is used as the radiation source 4. In this way, electromagnetic pulses having a pulse width as short as (down to) 1 ns can be achieved.

Further, a detection unit 7, 9 is integrated in the housing 2 and configured to detect ultrasonic waves 8 emanating from the object 3 in response to illuminating the object 3 with the time-varying electromagnetic radiation 5. The detection unit 7, 9 comprises at least one ultrasonic transducer 7, in particular a piezoelectric ultrasonic transducer (PUT) and/or micromachined ultrasonic transducer (MUT), preferably at least one capacitive micromachined ultrasonic transducer (CMUT) or piezoelectric micromachined ultrasonic transducer (PMUT), which has a front side 7a and a back side 7b opposite the front side 7a and is configured to convert ultrasonic waves 8 emanating from the object 3 and impinging at the front side 7a of the ultrasonic transducer 7 into detection signals S. A second electronic controller 9 is provided at the back side 7b of the ultrasonic transducer 7 and configured to process the detection signals, in particular to digitize and/or filter the detection signals and to reconstruct an image of the object 3 based on the digitized and/or filtered detection signals.

Preferably, the radiation sources 4, in particular the VCSELs, of the illumination unit 4, 6, the MUTs 7 of the detection unit 7, 9, and the first and second electronic controller 6, 9 are provided and/or integrated on the same circuit board and/or on the same chip. Preferably, the first and second electronic controller 6, 9 comprise, respectively, integrated electronics as a laser controller (i.e., coded pulse, wavelength, pulse length), OA/US mode controller, power supply, computing unit (computing on a chip) with basic functionality (i.e., signal acquisition, signal digitalization, signal processing, filtering and denoising, image formation), wireless communication unit, and/or memory circuitry.

Preferably, the device 1 can be provided with a laser safety unit for tissue contact detection. For this purpose, at least one sensor unit 10 is integrated in the housing 2 and configured to detect whether the handheld device 1 is in contact with and/or close to the object 3, wherein the first electronic controller 6 is configured to control the at least one radiation source 4 to emit the pulses of electromagnetic radiation 5 only if the at least one sensor unit 10 detects that the handheld device 1 is in contact with or close to the object 3. Alternatively or additionally, the at least one radiation source 4 is controlled so as to stop emitting pulses of the electromagnetic radiation 5, if the at least one sensor unit 10 detects that the handheld device 1 is not in contact with or not close to the object 3.

The at least one sensor unit 10 may be or may comprise a stray light sensor, such as a photodiode, which is configured to detect stray light 11 emanating from the object 3 in response to illuminating the object 3 with the time-varying electromagnetic radiation 5, wherein detecting the stray light 11 is indicative that the handheld device 1 is in contact with and/or close to the object 3, whereas not detecting stray light 11 is indicative that the handheld device 1 is not in contact with and/or not close to the object 3. Alternatively or additionally, the at least one sensor unit 10 may comprise and/or maybe configured as a contact sensor which is configured to detect whether the handheld device 1 is in contact with the object 3. For example, the contact sensor may be an optical sensor which is configured to detect electromagnetic radiation, such as stray light or reflected light, which is indicative of the presence of the object 3 at and/or close to the device 1. Alternatively or additionally, the contact sensor may be a conductivity sensor having an electrical conductivity which is dependent on whether it is in contact with (or close to) the object 3 or not. Alternatively, the contact sensor may be configured such that its electrical resistance and/or capacity depends on whether the sensor is in contact with or close to the object 3 or not. Alternatively or additionally, the contact sensor may be a thermocouple configured to generate an electrical voltage which is dependent on the temperature which in turn depends on whether the contact sensor is in contact with the object 3 or not. Alternatively or additionally, the contact sensor may be an ultrasonic sensor which is configured to emit and detect ultrasonic waves, wherein detecting ultrasonic waves (e.g. pulse-echo ultrasonic waves) reflected by the object 3 and having a certain intensity is indicative that the device 1 is in contact with or close to the object 3. Alternatively or additionally, the contact sensor may be an accelerometer sensor configured to detect an acceleration and/or deceleration of the device 1 based on which a position of the device 1 relative to the object 3 can be derived and/or estimated. Last but not least, the contact sensor may, alternatively or additionally, be a piezoelectric sensor which is configured to generate an electrical voltage depending on a mechanical pressure exerted on the piezoelectric sensor due to a (possible) contact between device 1 and object 3.

The first electronic controller 6 is preferably configured to control the at least one radiation source 4 to emit the pulses of electromagnetic radiation 5 only if the stray light sensor or contact sensor (according to one of the above-mentioned types of contact sensors) provides a signal which is indicative that the handheld device 1 is in contact with or close to the object 3.

The at least one sensor unit 10 preferably comprises both a stray light sensor and a contact sensor (preferably according to one of the above-mentioned types of contact sensors), and the first electronic controller 6 is configured to control the radiation source 4, in particular the VCSEL(s), to emit the pulses of electromagnetic radiation 5 only if both the stray light sensor and the contact sensor detect that the handheld device 1 is in contact with and/or close to the object 3, and/or to stop emitting pulses of electromagnetic radiation 5 if at least one of the stray light sensor and/or the contact sensor detects that the handheld device 1 is not in contact with or not close to the object 3.

Preferably, a calibration unit 12, 13, 15 is integrated in the housing 2 and comprises at least one calibration sensor 13, 15 which is configured to detect a reference signal which is indicative of an intensity of the electromagnetic radiation 5 emitted by the illumination unit 4, 6 and/or indicative of a sensitivity of the detection unit 7, 9 with respect to ultrasonic waves. For this purpose, the calibration unit 12, 13, 15 is preferably configured to measure the optical and/or acoustic path (i.e., optical emitter and/or optical receiver or, respectively, acoustic receiver), phantoms with optical and acoustic targets, and/or optical and acoustic targets positioned directly to the handheld device 1.

For example, calibration sensor 13 comprises an optical sensor, such as a photodiode, which detects electromagnetic radiation 14 which is reflected and/or scattered by the imaged object 3 and/or by a reference object (not shown) in response to illuminating the object 3 with the pulsed electromagnetic radiation 5. Preferably, calibration sensor 13 comprises and/or is given by a stray light sensor, in particular a photodiode, which is configured to detect, as the reference signal, stray light 14 emanating from the imaged object 3 and/or from the reference object in response to illuminating the imaged object 3 or reference object, respectively, with the electromagnetic radiation 5.

Alternatively or additionally, calibration sensor 15 comprises an ultrasound sensor which detects ultrasonic waves 16 emanating from the imaged object 3 and/or reference object in response to irradiating the object 3 with pulsed electromagnetic radiation 5. Preferably, the calibration sensor 15 comprises and/or is given by at least one of the ultrasonic transducer(s) 7 of the detection unit 7, 9 and configured to detect, as the reference signal, ultrasonic waves 16 emanating from the imaged object 3 and/or a reference object in response to illuminating the imaged object 3 or reference object, respectively, with the time-varying electromagnetic radiation 5. Preferably, a calibration controller 12 is configured to derive a calibration signal from the detected optical and/or acoustic reference signal(s). The first electronic controller 6 preferably controls the at least one radiation source 4 based on or by considering the calibration signal. Alternatively or additionally, the second electronic controller 9 is configured to control, based on or by considering the calibration signal, the sensitivity of at least one ultrasonic transducer 7 and/or the processing of the detection signals.

The housing 2 of the device 1 preferably includes an interface unit 17 which is configured to establish a wireless and/or wired data communication between the first and/or second electronic controller 6, 9 and a computer system 20, in particular a mobile computer device 21 and/or a computer workstation and/or a cloud computing system 22, which is located outside the housing 2 of the handheld device 1 and configured to further process the data of the image which has been reconstructed by the second controller 9.

Further, it is preferred that the ultrasonic transducer 7 has a frequency-dependent sensitivity to ultrasonic waves within a so-called transducer bandwidth, which includes a center frequency region in which the sensitivity to ultrasonic waves is maximum, and the first electronic controller 6 is configured to tune, by controlling the at least one radiation source 4, the pulse width of the emitted pulses of electromagnetic radiation 5 such that the resulting ultrasonic waves 8 emanating from the object 3 in response to illuminating the object 3 with the emitted pulses of electromagnetic radiation 5 exhibit at least one frequency bandwidth which matches with and/or falls within the transducer bandwidth. In other words, the laser repetition rate and pulse length are preferably tuned in agreement with the transducer central frequency for optimal tissue SNR and image resolution. This is further illustrated in the following.

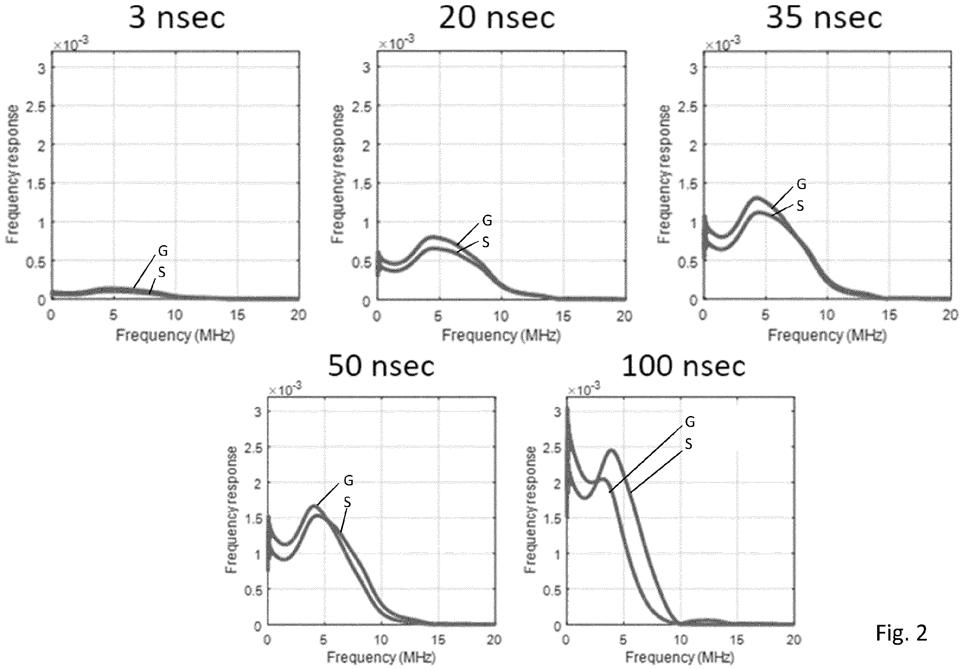

Figure 2 shows diagrams depicting a response ("frequency response") of the ultrasound transducer(s) 7 (having a center frequency at 6 MHz) of the detection unit 7, 9 over the frequency (given in MHz) of ultrasonic waves emanating from point-like optoacoustic sources having a size below the system resolution in response to an illumination with Gaussian (G) and square (S) shaped pulses of electromagnetic radiation exhibiting different pulse durations/widths of 3, 10, 35, 50 and 100 nsec (nanoseconds, ns). As apparent from the frequency responses obtained for 3 ns and 100 ns, by matching the pulse width of the illumination to the transducer bandwidth, the frequency response of the transducer 7 can be enhanced up to a factor of approximately 10.

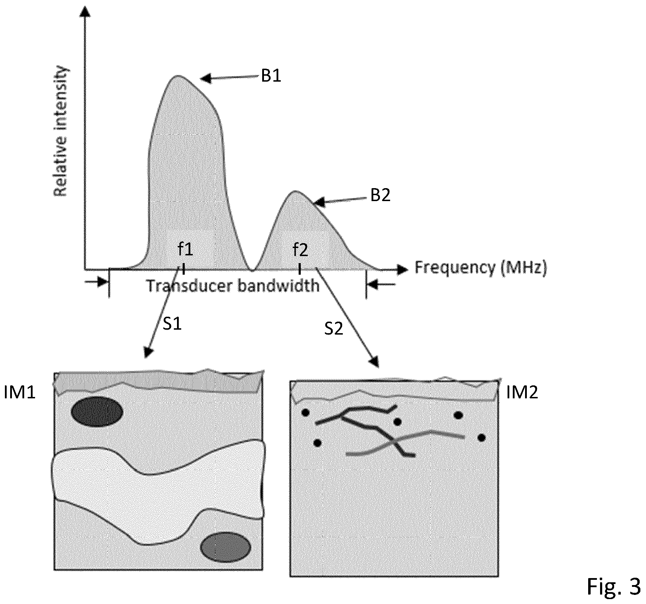

Another preferred embodiment, which is also referred to as "second harmonic imaging", is exemplarily explained with reference to figure 3, which shows a diagram (upper part) depicting a response ("Relative intensity") of an ultrasound transducer 7 (having a transducer bandwidth) of the detection unit 7, 9 over the frequency of ultrasonic waves emanating from an object, and optoacoustic images IM1, IM2 (lower part) reconstructed from the responses of a plurality of such ultrasound transducers 7.

In this embodiment, ultrasonic waves 8 emanating from the object 3 in response to illuminating the object 3 with the pulses of electromagnetic radiation 5 exhibit a first frequency bandwidth B1 at and/or around a fundamental frequency f1, and a second frequency bandwidth B2 at and/or around a second harmonic frequency f2 of the fundamental frequency f1 (f2 = 2 × f1). Preferably, the transducer bandwidth of the transducers 7 of the detection unit 7, 9 is configured and/or configurable to be sensitive to both at least a part of the first frequency bandwidth B1 at the fundamental frequency f1 and at least a part of the second frequency bandwidth B2 at the second harmonic frequency f1. In the example illustrated in figure 3, the first frequency bandwidth B1 and second frequency bandwidth B2 are completely (B1) or almost completely (B2) within the transducer bandwidth.

Similarly (and alternatively or additionally to choosing, configuring and/or controlling the sensitivity of the ultrasonic transducer(s)), the first electronic controller 6 may be configured to tune, preferably by controlling the at least one radiation source 4, the pulse width of the emitted pulses of electromagnetic radiation 5 such that the resulting ultrasonic waves 8 exhibit a first frequency bandwidth B1 at a fundamental frequency f1 and a second frequency bandwidth B2 at a second harmonic frequency f2 of the fundamental frequency f1 (f2 = 2 × f1), wherein both the fundamental frequency bandwidth B1 and the second harmonic frequency bandwidth B2 match with and/or fall within the transducer bandwidth. The above explanations with reference to figure 3 (upper part) apply accordingly.

Preferably, the second electronic controller 9 is configured to separate, preferably by means of at least one filter such as a bandpass filter, a low-pass filter and/or a high-pass filter, the detection signals into first detection signals S1 and second detection signals S2, wherein the first detection signals S1 characterize the detected ultrasonic waves exhibiting the first frequency bandwidth B1 at and/or around the fundamental frequency f1, and the second detection signals S2 characterize the detected ultrasonic waves exhibiting the second frequency bandwidth B2 at the second harmonic frequency f2. Further, the integrated second electronic controller 9 and/or the external computer system 20 is configured to separately process the first detection signals S1 and second detection signals S2, in particular by reconstructing two different images IM1, IM2 of the object 3 based on the first and second detection signals S1, S2, respectively. As schematically and exemplarily illustrated in figure 3 (lower part), the different images IM1, IM2 obtained in this way reveal different kinds of image information regarding the object to be imaged. In the present example, the first image IM1 which is based on the first frequency bandwidth B1 (also referred to as "first harmonic") is a bulky image of the object, whereas the second image IM2 which is based on the second frequency bandwidth B2 (also referred to as "second harmonic") greatly enhances the details and has a greater spatial resolution due to the contribution of high frequencies.

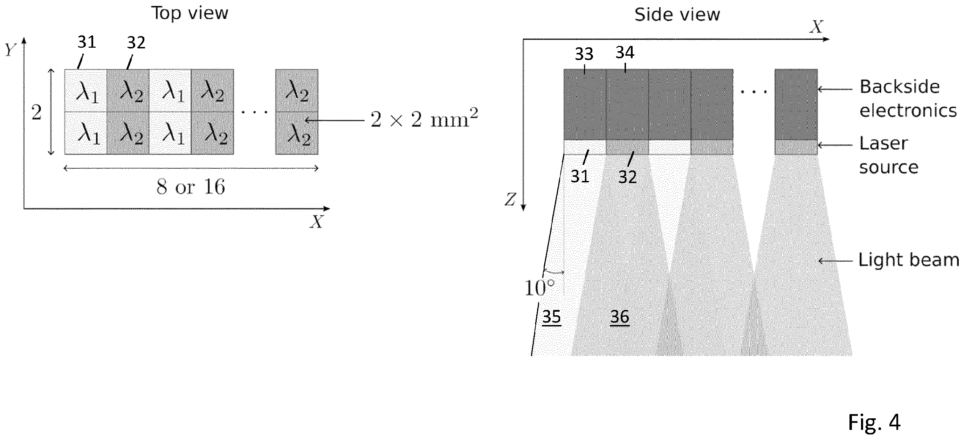

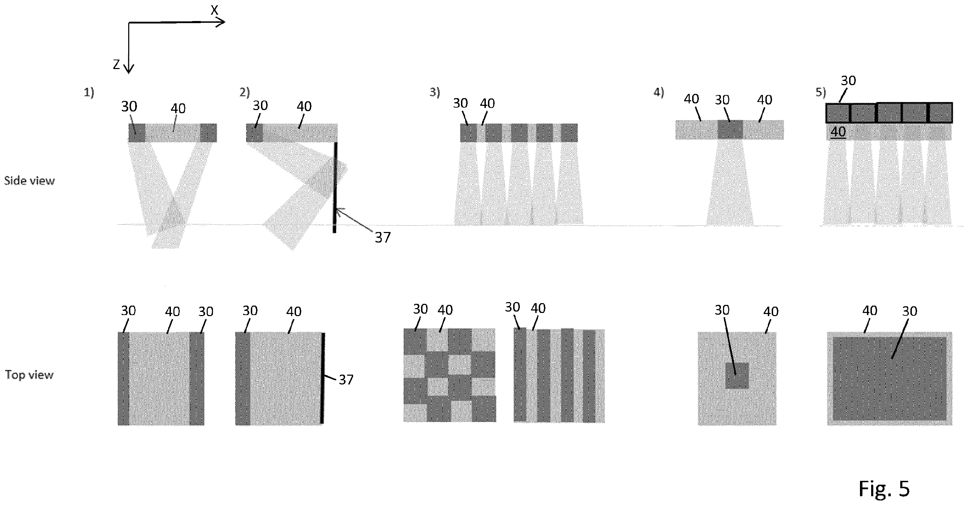

Preferably, the illumination unit 4, 6 comprises a plurality of vertical-cavity surface-emitting lasers (VCSELs) and/or the detection unit 7, 9 comprises a plurality of ultrasonic transducers, in particular piezoelectric ultrasonic transducers (PUTs) and/or micromachined ultrasonic transducers 7 (MUTs), in particular capacitive micromachined ultrasonic transducers (CMUTs) and/or piezoelectric micromachined ultrasonic transducers (PMUTs). In the following, particularly preferred arrangements and/or features of the VCSELs and/or PUT(s) or MUTs, respectively, are described in more detail with reference to figures 4 and 5.

Figure 4 shows a top view (left part) and side view (right part) of an example of a two-dimensional arrangement of VCSELs 31, 32 in an array or grid comprising 2×8 or 2×16 VCSELs in Y-X-plane. In the given example, two different types of VCSELs 31, 32 emitting electromagnetic radiation at different wavelengths λ₁, λ₂ (or different wavelength ranges or spectra) are arranged alternately in X direction, wherein two VCSELs 31 emitting a first wavelength λ₁ alternate with two VCSELs 32 emitting a second wavelength λ₂. For example, the VCSELs 31, 33 are configured to emit at least one of the following wavelengths: 1060 nm, 960 nm, 850 nm, 800 nm, 760 nm, 720 nm, 680 nm. In alternative examples (not shown), other numbers and/or arrangements of VCSELs are possible, for example from 1×1 (e.g. for endoscope applications) to 4×64 (256-channel) and/or more than two different VCSELs emitting more than two different wavelengths can be mixed.

In the present example, the VCSELs 31, 32 emit the electromagnetic radiation at the front side which has a size of 2×2 mm². Alternatively, the front side of the VCSELs 31, 32 can have other sizes, for example 1×1 mm², 1×2 mm², 2.5×2.5 mm².

In an alternative embodiment (not shown), it is also possible to arrange the VCSELs in a one-dimensional array along a straight line or along an arbitrary line and/or to provide only one type of VCSELs emitting electromagnetic radiation at only one wavelength or wavelength range or spectrum. Further, it is possible to arrange the VCSELs in an optimized random distribution.

As apparent from the side view shown in figure 4, each of the VCSELs 31, 32 is provided with an electronic controller 33, 34, which is also referred to as "first electronic controller" (cf. figure 1) and provided at the back side of the respective VCSEL 31, 32.

As further shown in the side view, each VCSEL 31, 32 is preferably configured to emit the electromagnetic radiation in a, preferably slightly, divergent light beam or light cone 35, 36 having an aperture angle of approximately 10° at each side (i.e. 20° in total). Alternatively, smaller or larger aperture angles, for example 5° or 15°, can be preferred, depending on the specific arrangement of the VCSELs and/or preferred application of the device or system. Alternatively or additionally, beam shaping can be achieved, for example, by means of micro lens(es) and/or cylindrical lens(es) configured to bend the beam for flat-top illumination. Alternatively, it is also possible to provide no lenses at all and/or to provide diffusers for some applications.

Preferably, the VCSELs 31, 32 are controllable and/or tunable to emit pulses of electromagnetic radiation exhibiting a pulse length/width from 1 to 100 ns and/or a repetition rate from 100Hz to 10 kHz and/or an energy/puls per unit from 1-20µJ/ mm². Further, it may be preferred that the pulses are coded with frequencies from 10 Hz to 100 kHz for any pulse sequence.

Although not explicitly shown in figure 4, at least one sensor unit 10 (see figure 1), such as a photodiode, can be provided (e.g. next to and/or close to at least one of the VCSELs 31, 32 and/or next to and/or close to the array of VCSELs 31, 32) as a stray light sensor which is configured to detect stray light 11 (see figure 1) emanating from the object (not shown) in response to illuminating the object with the light beam(s).

Figure 5 shows a side view (upper part) and top view (lower part) of examples 1) to 5) of different arrangements of VCSELs 30 and MUTs 40, wherein VCSELs 30 are represented by dark areas, while MUTs 40 are represented by bright areas.

In general, the total number of MUTs 40 may vary depending on the geometry and layout of the detection unit and/or on the purpose and/or application of the device. For example, the total number of MUTs 40 may be between 128 and 512 for imaging applications, and between 1 and 128 for patches (which can be applied, e.g., onto the skin of a patient) or for endoscopes. Further, the sensitive area and/or the front side of a single MUT 40 may have a size of approximately 1×1 mm. Further, the central frequency of a MUT 40 and/or second harmonic may be between 50 kHz and 10 MHz (OA macroscopy), between 10 and 50 MHz (OA mesoscopy), or between 30 and 120 MHz (OA microscopy). Preferably, the frequency bandwidth, also referred to as "transducer bandwidth", is broadband: The percent bandwidth, defined as the ratio between the transducer bandwidth and its central frequency, is superior to 100% for OA and superior to 50% for US. Notwithstanding the examples shown in figure 5 and described in more detail below, the layout and/or spatial arrangement of the MUTs can be concave, linear (i.e., rectangular or square), circle, oval, 2D, 3D, single element, geometrically focused, mechanically focused (i.e., without acoustic lenses) and/or mounted on a flex electronics. In embodiments where the MUTs are not only used as receivers for optoacoustic waves (OA imaging), but also as transmitters of ultrasonic waves (US imaging), the pulses of the transmitted ultrasonic waves have pulse lengths preferably between 1 and 100 ns and/or a repetition rate between 100 Hz and 10 kHz.

As apparent from examples 1) and 2), the VCSELs 30 can be provided at both sides of an array of MUTs 40 (wrt. an elevational direction X of the MUT array) or, respectively, at only one side of an array of MUTs 40.

Further, as already discussed in detail above, the VCSELs' 30 pulse length can be preferably tuned to match the MUTs' 40 receiving sensitivity (bandwidth), i.e. the width of the laser pulse(s) can be optimized to make the object's 3 response (i.e. OA waves) fit to the MUTs' 40 bandwidth to achieve maximum detection signals.

Further, as apparent from example 1), the VCSELs 30 can be configured and/or arranged to deliver the electromagnetic radiation obliquely at any angle (configuration of dark-field illumination).

As illustrated in example 2), an optical mirror 37 and/or lense(s) (not shown) can be provided to redirect the beam of electromagnetic radiation emitted by the VCSELs 30 on the MUTs' principal acoustic axis (configuration of bright field illumination).

As illustrated in example 3), the MUTs 40 and VCSELs 30 can be arranged on the same plane in a matrix and in a specific, e.g. alternating and/or regular such as checkered or striped, pattern (configuration of semi-bright field illumination).

As illustrated in example 4), the VCSELs 30 are centered in the array of MUTs 40, which corresponds to a bright field illumination and is also referred to as "through-hole illumination".

In example 5), a two-dimensional array of VCSELs 30 is coaxially aligned with a two-dimensional array of optically transparent MUTs 40, so that the illumination of the object occurs through the MUTs 40 (configuration of bright field illumination).

Although not explicitly shown in figure 5, at least one sensor unit 10 (see figure 1), such as a photodiode, can be provided (e.g. next to and/or close to at least one of the VCSEL 30) as a stray light sensor which is configured to detect stray light 11 (see figure 1) emanating from the object (not shown) in response to illuminating the object with the light beam(s).

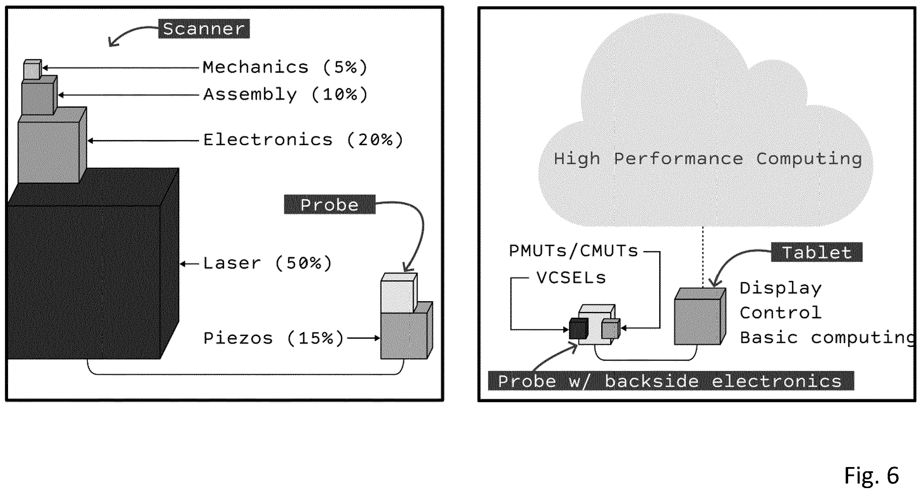

Figure 6 shows a comparison of a system for optoacoustic imaging according to the prior art (left) with a system according to the present disclosure (right) in a schematic representation. As apparent from the figure, the concept according to present disclosure allows for reducing the bulkiness/size, costs and/or power consumption of conventional optoacoustic scanners, in particular by providing a handheld probe with PMUTs and/or CMUTs and VCSELs with integrated backside electronics for the PMUTs/CMUTs and VCSELs, an external computer, such as a tablet computer, for display, user control of the handheld probe and basic computing tasks, and (another) external computer system, in particular a computer workstation and/or cloud computing solution, which is configured for high performance computing.

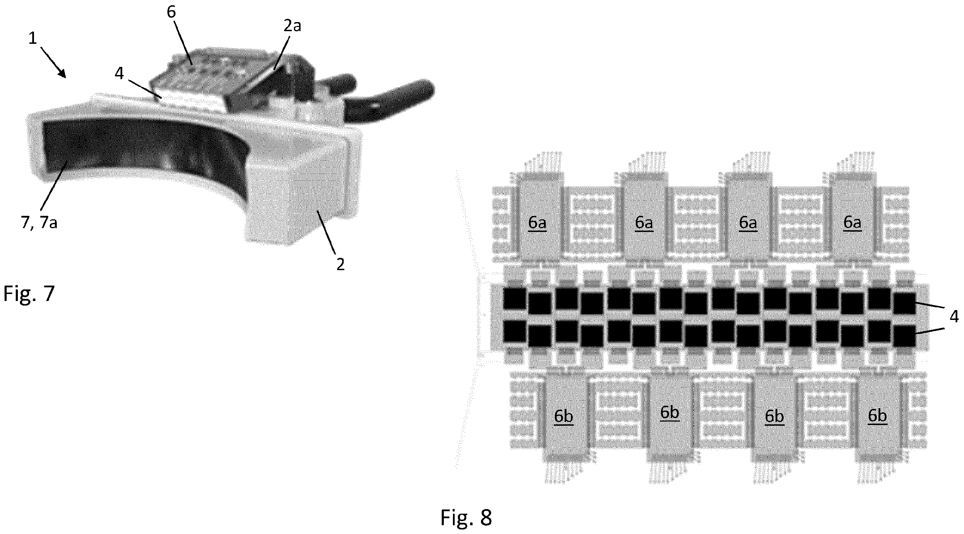

Figure 7 shows a perspective view of another example of a device 1 for optoacoustic imaging. The device 1 comprises a housing 2 having a size and shape such that it can be grasped by an operator's hand (not shown) and guided and/or positioned by the operator relative to an object (not shown) to be imaged. Although a "classic" bulk piezo implementation in conjunction with a VCSEL array is shown, the bulk piezo implementation can be replaced by MUT(s) and/or a MUT array in a miniaturized implementation.

In the present example, an illumination unit 2a, 4, 6 is provided on the housing 2, preferably attached to and/or mounted on an outer surface of the housing 2 for illuminating the object with electromagnetic radiation. The illumination unit 2a, 4, 6 comprises an array of VCSELs 4 provided on a front or top surface of a cuboidal or brick-shaped copper block 2a serving as a heat sink.

A first electronic controller 6, which is configured to control the VCSELs 4, is provided on a side or lateral surface, which runs perpendicularly to the front or top surface, of the copper block 2a. In this way, the first electronic controller 6 is provided close to the VCSELs 4 to ensure short distances, preferably of less than 10 mm (10-20ns), less than 500 µm (<1 ns), therebetween. Preferably, the VCSELs 4 are connected to the first electronic controller 6 via bonded gold wires to achieve both good electric coupling and good thermal management. Further, in this way, the VCSELs 6 can be controlled by the first electronic controller 6 to emit pulses of electromagnetic radiation exhibiting short pulse widths (durations) as low as 10 ns to 20 ns.

This arrangement is particularly preferred, e.g., when several VCSEL arrays having a size of approx. 1 to 4 mm² are combined to a large illumination area as shown in the figure. To ensure an effective heat dissipation, the VCSEL submounts are provided on the copper block 2a (which may have one or more heat pipes to further improve heat dissipation) and the electronic controller 6 is provided in close proximity to the VCSELs 4. In this way, electromagnetic pulses having pulse widths as short as (down to) 10 or 20 ns can be achieved (10 mm wire bonding).

Further, a detection unit 7 is integrated in the housing 2 and configured to detect ultrasonic waves emanating from the object when being illuminated with electromagnetic radiation emerging from the VCSELs 4. Preferably, the detection unit 7 comprises a plurality of ultrasonic transducers 7a, in particular piezoelectric ultrasonic transducers (PUTs) and/or micromachined ultrasonic transducers (MUTs), preferably capacitive micromachined ultrasonic transducers (CMUTs) or piezoelectric micromachined ultrasonic transducers (PMUTs), which are arranged along a concave surface. A second electronic controller (not shown) is provided at the back side of the ultrasonic transducers 7a and configured to process detection signals, which are generated by the transducers 7a when detecting the ultrasonic waves emanating from the object, for example by digitizing and/or filtering the detection signals and/or reconstructing an image of the object based on the digitized and/or filtered detection signals.

Figure 8 shows a schematic representation of a circuit diagram of an illumination unit as exemplarily shown in Fig. 7. In the present example, a plurality of VCSELs 4 is arranged to form a 2 × 16 array. Alternatively, other forms and/or sizes of arrays are possible, for example a 1 × 16, 3 × 16, 4 × 16, 2 × 32 or 2 × 64 array. Further, alternatively to arranging the VCSELs 4 along a more or less linear array, it is possible to arrange the VCSELs 4 along a curved array.

In the example shown, the VCSELs 4 are alternately offset in pairs upwards and downwards in a direction perpendicular to the direction of the array, wherein the pairs offset upwards being wired with and controlled by upper first electronic controllers 6a and the pairs offset downwards being wired with and controlled by lower first electronic controllers 6b. Other than suggested by the schematic representation in the figure, the first electronic controllers 6a, 6b are preferably provided on a plane which is perpendicular to the plane defined by the array of the VCSELs 4, as exemplarily shown in Fig. 7.

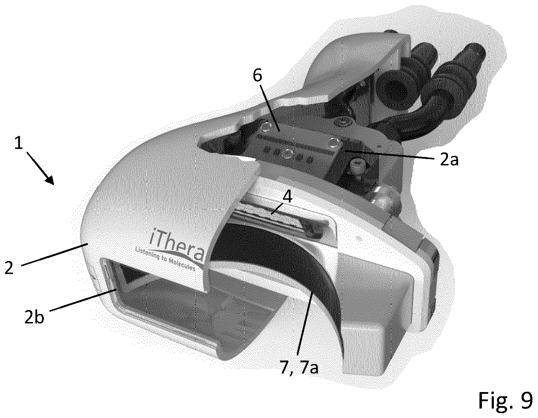

Figure 9 shows a perspective view of yet another example of a device 1 for optoacoustic imaging comprising a housing 2 having a size and shape such that it can be grasped by an operator's hand (not shown) and guided and/or positioned by the operator relative to an object (not shown) to be imaged.

In the present example, the housing 2 encloses both the illumination unit 2a, 4, 6 and the detection unit 7 corresponding to a (miniaturized) implementation of MUT(s) 7a and/or a MUT 7a array. For reasons of clarity, only a left part of the housing 2 is shown so that the components 2a, 4, 6 and 7 integrated in and surrounded by the housing 2 can be seen. At the front end of the housing 2, which is brought in contact with the object during imaging, an opening or window 2b is provided through which electromagnetic radiation emitted by the VCSELs 4 can pass through towards the object, and ultrasonic waves emanating from the object can pass through towards the detection unit 7. Regarding further preferred features and/or functionalities of the present example, the above explanations with reference to the examples shown in figures 7 and 8 apply accordingly to the example shown in figure 9.

## Claims

1. A handheld device (1) for optoacoustic imaging of an object (3), the device (1) comprising a housing (2) configured to be grasped by an operator's hand and guided and/or positioned by the operator relative to the object (3) during imaging, wherein the following components are integrated in and/or provided on the housing (2):
- an illumination unit (4, 6) configured to illuminate the object (3) with time-varying electromagnetic radiation (5), the illumination unit (4, 6) comprising at least one radiation source (4), in particular at least one laser diode (LD), vertical-cavity surface-emitting laser (VCSEL) or light-emitting diode (LED), having a front side (4a) and a back side (4b) opposite the front side (4a) and configured to emit the electromagnetic radiation (5) at the front side (4a) of the at least one radiation source (4), the illumination unit (4, 6) further comprising a first electronic controller (6) provided at the back side (4b) of and/or close to the at least one radiation source (4) and configured to control the at least one radiation source (4) to emit pulses of electromagnetic radiation (5) exhibiting at least one of a predefined electromagnetic spectrum and/or wavelength(s), pulse amplitude, pulse shape, pulse width (duration) and/or pulse repetition rate and/or pulse polarization and/or pulse phase, and
- a detection unit (7, 9) configured to detect ultrasonic waves (8) emanating from the object (3) in response to illuminating the object (3) with the time-varying electromagnetic radiation (5), the detection unit (7, 9) comprising at least one ultrasonic transducer (7), in particular at least one micromachined ultrasonic transducer (MUT) and/or at least one piezoelectric ultrasonic transducer (PUT), having a front side (7a) and a back side (7b) opposite the front side (7a) and configured to convert ultrasonic waves (8) impinging at the front side (7a) of the at least one ultrasonic transducer (7) into detection signals, the detection unit (7, 9) further comprising a second electronic controller (9) provided at the back side (7b) of the at least one ultrasonic transducer (7) and configured to process the detection signals, in particular to digitize and/or filter the detection signals and to reconstruct an image of the object (3) based on the digitized and/or filtered detection signals.

2. The handheld device (1) according to claim 1, wherein the at least one radiation source (4) is and/or comprises a vertical-cavity surface-emitting laser (VCSEL) and/or the at least one ultrasonic transducer (7) is a micromachined ultrasonic transducer (MUT), in particular a capacitive micromachined ultrasonic transducer (CMUT) and/or piezoelectric micromachined ultrasonic transducer (PMUT), and/or a piezoelectric ultrasonic transducer (PUT).

3. The handheld device (1) according to at least one of the preceding claims, wherein the first electronic controller (6) comprises an integrated circuit based on complementary metal-oxide-semiconductor (CMOS, Si or GaN) technology and is mounted, in particular by flip-chip bonding, on the back side (4b) of and/or close to the at least one radiation source (4) in such a way that electrical connections between the integrated circuit and the at least one radiation source (4) are
- shorter than 10 mm, in cases where the integrated circuit is mounted in a lateral region of the at least one radiation source (4), in particular on a lateral surface running perpendicularly to a top surface on which the at least one radiation source (4) is provided, or
- shorter than 500 µm, in cases where the integrated circuit is mounted on the back side (4b) of the at least one radiation source (4).

4. The handheld device (1) according to at least one of the preceding claims, wherein the first electronic controller (6) is configured to control the at least one radiation source (4) to emit pulses of electromagnetic radiation exhibiting a pulse width (duration) between 1 ns and 100 ns and/or a repetition rate between 100 Hz and 10 kHz.

5. The handheld device (1) according to at least one of the preceding claims, further comprising at least one sensor unit (10) configured to detect whether the handheld device (1) is in contact with and/or close to the object (3), wherein the first electronic controller (6) is configured to control the at least one radiation source (4) to emit the pulses of electromagnetic radiation (5) only if the at least one sensor unit (10) detects that the handheld device (1) is in contact with or close to the object (3), and/or to stop emitting pulses of electromagnetic radiation (5) if the at least one sensor unit (10) detects that the handheld device (1) is not in contact with or not close to the object (3), wherein the at least one sensor unit (10) optionally comprises
- a stray light sensor, in particular a photodiode, configured to detect stray light (11) emanating from the object (3) in response to illuminating the object (3) with the time-varying electromagnetic radiation (5), wherein detecting the stray light (11) is indicative that the handheld device (1) is in contact with and/or close to the object (3), whereas not detecting stray light (11) is indicative that the handheld device (3) is not in contact with and/or not close to the object (3), and/or
- at least one contact sensor configured to detect whether the handheld device (1) is in contact with the object (3), wherein the at least one contact sensor comprises at least one of the following: an optical sensor, a conductivity sensor, a thermocouple, an ultrasonic sensor, an accelerometer sensor, a resistive and/or capacitive touch screen and/or a piezoelectric sensor.

6. The handheld device (1) according to claim 5, wherein the at least one sensor unit (10) comprises both the stray light sensor and the contact sensor, and wherein the first electronic controller (6) is configured to control the at least one radiation source (4) to emit the pulses of electromagnetic radiation (5) only if both the stray light sensor and the contact sensor detect that the handheld device (1) is in contact with and/or close to the object (3), and/or to stop emitting pulses of electromagnetic radiation (5) if at least one of the stray light sensor and/or the contact sensor detects that the handheld device (1) is not in contact with or not close to the object (3).

7. The handheld device (1) according to at least one of the preceding claims, further comprising a calibration unit (12, 13, 15) comprising at least one calibration sensor (13, 15) configured to detect a reference signal, in particular electromagnetic radiation (14) reflected and/or scattered by the imaged object (3) and/or by a reference object and/or ultrasonic waves (16) emanating from the imaged object (3) and/or a reference object, and a calibration controller (12) configured to derive a calibration signal from the detected reference signal, wherein a) the first electronic controller (6) is configured to control the at least one radiation source (4) by considering the calibration signal and/or b) the second electronic controller (9) is configured to control the at least one ultrasonic transducer (7) and/or to process the detection signals by considering the calibration signal.

8. The handheld device (1) according to claim 7, wherein the at least one calibration sensor (13, 15) comprises and/or is given by a stray light sensor (13), in particular a photodiode, which is configured to detect, as the reference signal, stray light (14) emanating from the imaged object (3) and/or from a reference object in response to illuminating the imaged object (3) or reference object, respectively, with the time-varying electromagnetic radiation (5).

9. The handheld device (1) according to claim 7 or 8, wherein the at least one calibration sensor (13, 15) comprises and/or is given by at least one ultrasonic transducer (7, 15) of the detection unit (7, 9) and configured to detect, as the reference signal, ultrasonic waves (16) emanating from the imaged object (3) and/or a reference object in response to illuminating the imaged object (3) or reference object, respectively, with the time-varying electromagnetic radiation (5).

10. The handheld device (1) according to at least one of the preceding claims, further comprising an interface unit (17) configured to establish a data communication between the first and/or second electronic controller (6, 9) and a computer system (20), in particular a mobile computer device (21) and/or a computer workstation and/or a cloud computing system (22), located outside the handheld device (1) and configured to further process the reconstructed image.

11. The handheld device (1) according to at least one of the preceding claims, wherein
- the first electronic controller (6) is configured to control the at least one radiation source (4) to, in particular simultaneously, emit at least two different time series patterns of electromagnetic radiation (5), wherein the at least two time series patterns differ from each other with respect to at least one of the following: amplitude, shape, duration and/or repetition rate,
- the at least one ultrasonic transducer (7) is configured to detect the ultrasonic waves (8) emanating from the object (3) in response to, in particular simultaneously, illuminating the object (3) with the at least two different time series patterns of electromagnetic radiation (5), to convert the detected ultrasonic waves (8) into detection signals, and to separate the detection signals into at least two separate detection signals, the at least two separate detection signals characterizing the response of the object (3) to each of the at least two different time series patterns of electromagnetic radiation, and
- the second electronic controller (9) is configured to separately process the at least two separate detection signals, in particular to reconstruct at least two different images of the object based on the at least two separate detection signals.

12. The handheld device (1) according to at least one of the preceding claims, wherein
- the at least one ultrasonic transducer (7) has a frequency-dependent sensitivity having a, preferably broadband, transducer bandwidth within which the at least one ultrasonic transducer (7) is sensitive to ultrasonic waves, the transducer bandwidth preferably including a center frequency region in which the sensitivity to ultrasonic waves is maximum, and
- the first electronic controller (6) is configured to tune, by controlling the at least one radiation source (4), the pulse width of the emitted pulses of electromagnetic radiation (5) such that the resulting ultrasonic waves (8) emanating from the object (3) in response to illuminating the object (3) with the emitted pulses of electromagnetic radiation (5) exhibit at least one frequency bandwidth which matches with and/or falls within the transducer bandwidth.

13. The handheld device (1) according to claim 12, wherein a) the ultrasonic waves (8) emanating from the object (3) in response to illuminating the object (3) with the emitted pulses of electromagnetic radiation (5) exhibit a first frequency bandwidth (B1) at a fundamental frequency (f1) and a second frequency bandwidth (B2) at a, in particular second, harmonic frequency (f2) of the fundamental frequency (f1), wherein the transducer bandwidth is configured and/or configurable to be sensitive to both at least a part of the first frequency bandwidth (B1) at the fundamental frequency (f1) and at least a part of the second frequency bandwidth (B2) at the, in particular second, harmonic frequency (f2) of the fundamental frequency (f1), and/or b) the first electronic controller (6) is configured to tune, by controlling the at least one radiation source (4), the pulse width of the emitted pulses of electromagnetic radiation (5) such that the resulting ultrasonic waves (8) emanating from the object (3) in response to illuminating the object (3) with the emitted pulses of electromagnetic radiation (5) exhibit a first frequency bandwidth (B1) at a fundamental frequency (f1) and a second frequency bandwidth (B2) at a, in particular second, harmonic frequency (f2) of the fundamental frequency (f1), wherein both the first frequency bandwidth (B1) and/or fundamental frequency (f1) and the second frequency bandwidth (B2) and/or harmonic frequency (f2) match with and/or fall within the transducer bandwidth of the ultrasonic transducer (7), and wherein
- the device (1) further comprises at least one filter, in particular bandpass filter, configured to separate the detection signals (S1, S2), which were obtained by detecting the ultrasonic waves (8) emanating from the object (3) in response to illuminating the object (3) with the emitted pulses of electromagnetic radiation (5) exhibiting the first frequency bandwidth (B1) at the fundamental frequency (f1) and the second frequency bandwidth (B2) at the harmonic frequency (f2), into first detection signals (S1) characterizing the ultrasonic waves (8) exhibiting the first frequency bandwidth (B1) at the fundamental frequency (f1), and second detection signals (S2) characterizing the ultrasonic waves (8) exhibiting the second frequency bandwidth (B2) at the harmonic frequency (f2), and
- the second electronic controller (9) is configured to separately process the first and second detection signals (S1, S2), in particular to reconstruct two different images (IM1, IM2) of the object (3) based on the first and the second detection signals (S1, S2).

14. The handheld device (1) according to at least one of the preceding claims, wherein the illumination unit (4, 6) comprises a plurality of vertical-cavity surface-emitting lasers (VCSELs) (30, 31, 32) and/or the detection unit (7, 9) comprises a plurality of ultrasonic transducers (40), in particular capacitive micromachined ultrasonic transducers (CMUTs) and/or piezoelectric micromachined ultrasonic transducers (PMUTs) and/or piezoelectric ultrasonic transducers (PUTs), and wherein at least one of the following applies:
- the VCSELs (30, 31, 32) are arranged in at least one one-dimensional or two dimensional array, in particular the VCSELs (30, 31, 32) can be arranged in any arbitrary line or grid,
- the VCSELs (30, 31, 32) are configured to emit electromagnetic radiation exhibiting only one electromagnetic spectrum and/or wavelength or to emit electromagnetic radiation exhibiting at least two, in particular only two or only three, different electromagnetic spectra and/or wavelengths,
- the VCSELs (30, 31, 32) are arranged on one side or both sides of the MUTs (40) in an elevational direction (X),
- the VCSELs (30, 31, 32) are configured and/or arranged to deliver the electromagnetic radiation obliquely at any angle (dark field illumination),
- an optical mirror (37) and/or lenses is or are provided to redirect the beam of electromagnetic radiation on the MUTs' (40) principal acoustic axis (Z) (bright field illumination),
- the MUTs (40) and VCSELs (30, 31, 32) are arranged on the same plane in a matrix with a specific regular pattern (configuration of semi-bright field illumination),
- the VCSELs (30, 31, 32) are centered in the MUTs (40) (through-hole illumination or bright field illumination),
- in case of transparent MUTs (40), VCSELs (30, 31, 32) are coaxially aligned with the MUTs (40), so that the illumination of the object (3) occurs via and/or through the MUTs (40) (bright field illumination).

15. A system for optoacoustic imaging of an object, the system comprising:
- a handheld device (1) according to at least one of the preceding claims and
- a computer system (20), in particular a mobile computer device (21) and/or a computer workstation and/or a cloud computing system (22), located outside the handheld device (1), in particular outside the housing (2) of the handheld device (1), and being in a data communication with the first and/or second electronic controller (6, 9), the computer system (20) being configured to further process the reconstructed image.
